(19) **Europäisches Patentamt / European Patent Office / Office européen des brevets**

(11) **EP 0 871 434 B2**

(12) **NEW EUROPEAN PATENT SPECIFICATION**

(45) Date of publication and mention
of the opposition decision:
**12.12.2007 Bulletin 2007/50**

(45) Mention of the grant of the patent:
**19.09.2001 Bulletin 2001/38**

(21) Application number: **96943884.5**

(22) Date of filing: **23.12.1996**

(51) Int Cl.:
**A61K 9/16** (2006.01)       **A61K 31/60** (2006.01)

(86) International application number:
**PCT/DK1996/000551**

(87) International publication number:
**WO 1997/023199 (03.07.1997 Gazette 1997/29)**

(54) **MODIFIED RELEASE ORAL PHARMACEUTICAL COMPOSITION CONTAINING 5-ASA AND METHOD FOR THE TREATMENT OF BOWEL DISEASES**

5-ASA ENTHALTENDES ORALES ARZNEIMITTEL MIT MODIFIZIERTER FREISETZUNG SOWIE VERFAHREN ZUR BEHANDLUNG VON DARMERKRANKUNGEN

COMPOSITION PHARMACEUTIQUE ORALE A LIBERATION MODIFIEE CONTENANT 5-ASA ET PROCEDE DE TRAITEMENT DE MALADIES INTESTINALES

(84) Designated Contracting States:
**AT BE CH DE DK ES FI FR GB GR IE IT LI LU MC NL PT SE**
Designated Extension States:
**AL LT LV RO SI**

(30) Priority: **21.12.1995 US 575954**

(43) Date of publication of application:
**21.10.1998 Bulletin 1998/43**

(73) Proprietor: **FARMACEUTISK LABORATORIUM FERRING A/S**
**DK-2720 Vanlöse (DK)**

(72) Inventors:
• **JEPSEN, Svenn, Klüver**
**DK-2840 Holte (DK)**
• **HALSKOV, Sören**
**DK-2830 Virum (DK)**

(74) Representative: **HOFFMANN EITLE**
**Patent- und Rechtsanwälte**
**Arabellastrasse 4**
**81925 München (DE)**

(56) References cited:
**EP-A- 0 148 811        EP-A- 0 453 001**
**WO-A-91/07949        US-A- 4 496 553**
**US-A- 4 980 173**

• **"Prescribing Information as of May 1993" zu "Pentasa" mesalamine controlled-release capsules.**
• **"Prescribing Information as of August 1995"(mesalamine) controlled-release capsules.**

• **"Prescribing Information as of December 1995" zu "Pentasa" (mesalamine) controlled-release capsules.**
• **"Prescribing Information as of June 1999" zu "Pentasa(mesalamine) controlled-release capsules.**
• **Drugs and the Pharmaceutical Sciences, Volume 37,Pharmaceutical Pelletization Technology, edited by Isaac Ghebre-Sellassie,Marcel Dekker, Inc., New York, 1989,Seiten 1,227 und 235.**
• **K.H.Bauer et al.Pharmazeutische Technologie, Georg Thieme Verlag, Stuttgart, New York, 1986, Seite 158.**
• **21 CFR Ch.1(4-1-02 Edition) 207.35.**
• **H.Sucker, et al., Pharm. Technologie, George Thieme Verlag,1991, S.377-379**
• **K.H.Bauer, et , al., Pharm. Technologie, Georg Thieme Verlag, 1986, S.358f**
• **H.Sucker, et al., Pharm. Technologie, Georg Thieme Verlag,1991, S.272-283**
• **Pentasa, September 1992**
• **DMW 1995, 120.Jg.,Nr.21,S.22**
• **I.R.Wilding,et al.,Aliment.Pharmacol.Ther. 2000.; 14:163-169**
• **L.Hellén, et al., Int.Journal of Pharmaceutics, 96 (1993) 205-216**
• **Rote Liste, 2004 "60274"**
• **Remington's Pharmaceutical Sciences, 18th Edition, Mack Publ. Company, 1990,P.1665**
• **Photograph of intermediary microcapsules used for making Pentasa-Retardtabletten.**
• **Remington's Pharmaceutical Sciences, 19th Edition, Mack Publ. Company, 1990,p.1627**
• **Webster's Third New Int. Dictionary, 1993,"Pellet"**

EP 0 871 434 B2

**(Cont. next page)**

- "Prescribing Information as of May 1993" zu "Pentasa" mesalamine controlled-release capsules.
- "Prescribing Information as of August 1995"(mesalamine) controlled-release capsules.
- "Prescribing Information as of December 1995" zu "Pentasa" (mesalamine) controlled-release capsules.
- "Prescribing Information as of June 1999" zu "Pentasa(mesalamine) controlled-release capsules.
- Drugs and the Pharmaceutical Sciences, Volume 37,Pharmaceutical Pelletization Technology, edited by Isaac Ghebre-Sellassie,Marcel Dekker, Inc., New York, 1989,Seiten 1,227 und 235.
- K.H.Bauer et al.Pharmazeutische Technologie, Georg Thieme Verlag, Stuttgart, New York, 1986, Seite 158.
- H.Sucker, et al., Pharm. Technologie, George Thieme Verlag,1991, S.377-379
- K.H.Bauer, et , al., Pharm. Technologie, Georg Thieme Verlag, 1986, S.358f
- H.Sucker, et al., Pharm. Technologie, Georg Thieme Verlag,1991, S.272-283
- L.Hellén, et al., Int.Journal of Pharmaceutics, 96 (1993) 205-216
- Remington's Pharmaceutical Sciences, 18th Edition, Mack Publ. Company, 1990,P.1665
- Photograph of intermediary microcapsules used for making Pentasa-Retardtabletten.
- Remington's Pharmaceutical Sciences, 19th Edition, Mack Publ. Company, 1990,p.1627

## Description

## FIELD OF THE INVENTION

The present invention provides improved oral pharmaceutical compositions for the treatment of inflammatory bowel diseases (IBD) such as Crohn's disease, Colitis Ulcerosa and related diseases, e.g. an unclassifiable form of said diseases or a diagnosed subtype of one of said diseases. The invention also provides a method for the treatment of IBD.

The composition of the present invention comprises as active ingredient 5-aminosalicylic acid (5-ASA) or pharmaceutically acceptable salts or esters thereof and is adapted for modified and targeted release of said 5-ASA in the diseased parts of the intestine, so as to obtain an advantageous and clinically important release and effect profile of 5-ASA. Thus, said administration form and release are improved compared to known therapy regimens.

## BACKGROUND OF THE INVENTION

The composition of the invention is individually coated granules adapted for oral administration as such, i. e. the composition is a "granulate" composition ready for use. The granule composition of the invention is an advantageous administration form in many clinical situations, e.g. with respect to patient having difficulties in swallowing and with respect to children not wanting to swallow tablets.

A further advantage is that the granules of the invention may be packaged in unit dosage forms comprising larger amounts of active 5-ASA, e.g. in sachets or sticks.

In principle, there is, in contrast to the maximal content of tablets and capsules, no upper limit to the amount of active ingredients in a unit dosage form of the composition according to the invention.

Thus, an advantage of the granule composition of the present invention is that it enables improved compliance values with respect to the therapy regimen, a clinically important parameter for the treatment of chronic diseases.

Overall, it should be noted that the question of a satisfactory compliance is especially important in the case of IBD, since failure to respond to medical treatment in many cases necessitates surgery, with the standard surgical operation in the treatment of ulcerative colitis in many cases being total proctocolectomy (removal of the colon and the rectum).

US patents 4,496,553 and 4,980,173 (Halskov) provide a method for the treatment of IBD by oral administration of 5-ASA compositions consisting essentially of free 5-ASA and carriers which will control the release of an effective amount of 5-ASA.

However, as opposed to the present invention, no mention of the administration of 5-ASA granules as such was disclosed, and the compositions described for clinical use were all in the form of tablets. The disclosure of said US patents, including the examples, is totally silent about the provision of a specific type of granule composition for direct oral intake. Nowhere in said patent specification is it suggested to develop or administer a granule composition.

Thus, in the examples of the above US patents, preparations of granulates pressed to form tablets with a diameter of 13.5 mm and a weight of 650 mg/tablet containing 250 mg of 5-ASA. The resulting tablets were used in clinical tests.

In the examples of the US patents, two intermediary preparations of granulates were described, one of them comprising 5-ASA, and the other being a "helper" granulate without 5-ASA, said "helper" granulate being prepared and admixed in order to facilitate the tablet compression involving the addition of talc and a lubricant mixture.

The '173 patent more specifically claims a method for the preparation of sustained-release tablets, useful for the treatment of colitis ulcerosa or Crohn's disease, comprising the steps of

a) preparing a first granulate from 5-ASA or a pharmaceutically acceptable salt or ester thereof and about 10% by weight (solids content based on the 5-ASA) of polyvinylpyrrolidone in an organic solvent thereby to provide granules of a particle size from about 0.7 to 1 mm, upon evaporation of the solvent.

b) applying onto said granules a coating composition, comprising a solution in an organic solvent of a pharmaceutically acceptable coating material which will gradually release the active ingredient upon arrival at the small intestine, thereby to provide coated granules upon evaporation of the solvent,

c) mixing the first granulate with about 5% by weight, calculated on the total solids content, of a lubricant and a conventional pharmaceutical tablet carrier in an amount in accordance with the desired size and active ingredient content of the tablet, and

d) forming tablets from the resulting mixture

Preferably the coating material is a cellulose derivative.

Neither of US 4,980,173 and US 4,496,553 disclose the use of a spheronization aid.

International application WO 94/28911 describes i.a. oral pharmaceutical compositions having pH regulating effect, in particular for raising a subnormal pH in the intestine, comprising a coated pH regulated alkaline material, preferably calcium carbonate. The composition may be formulated as entero-coated granules or tablets. The composition may further comprise a therapeutically active ingredient, e.g. 5-ASA. Such compositions may be formulated as combination granulates, where the 5-ASA is coated as described above with reference to US patent no. 4,496,553 or as combination tablets.

From international application WO 91/07949, a delayed release composition is known, which composition comprises an active compound and amorphous amylose. The active compound may be 5-ASA, cf. Example 4, a) of WO 91/07949. Such granules have a coating of amylose and ethyl cellulose, or of ethyl cellulose alone. The latter is used for comparison only and is not part of the claimed subject-matter of WO 91/07949. It appears from Example 4, a) that the 5-ASA-containing granules have a sieve value of from 1.40 to 1.70 mm. Thus, the granules disclosed in WO 91/07949 have sieve values different from the granules of the present invention

The specific requirements to the 5-ASA release properties of the granule composition as identified by the present inventors and defined by the present invention, were nowhere described or suggested in said US patents or WO 94/28911, let alone any hint or guidance as to how to arrive at the specific embodiments of the invention, said embodiments solving the problems identified and thus providing advantages in a non-predictable way.

## SUMMARY OF THE INVENTION

Surprisingly, according to the present invention, particular geometrical shapes of each of the granules in combination with the choice of and mixing of particular types of helper ingredients, provide granules with an especially advantageous and clinically important 5-ASA gastro-intestinal release.

Surprisingly, the granule composition of the present invention provides an advantageous release profile securing a clinically important bio-availability. Such a useful bio-availability is obtained due to the following characteristics: only a minor release of 5-ASA in the stomach is obtained, whereas a considerable amount of 5-ASA is available for an appropriate period of time in the small intestine, and also a considerably amount of 5-ASA is available in the large intestine.

Thus, in one of its main aspects, the invention provides a composition for oral administration, said composition being:

an oral modified release composition ensuring bioavailability of said 5-ASA in both the small and large intestine, and consisting of:

individually coated granules, each granule comprising:

- a core consisting essentially of 5-aminosalicylic acid (5-ASA) or a pharmaceutically acceptable salt or ester thereof and a physiologically acceptable spheronization aid, preferably a cellulose derivative, in particular microcrystalline cellulose, and

- a coating confining said core, said coating comprising a rate-limiting barrier material, which is a diffusion-rate limiting barrier material or an erodable, degradable rate-limiting barrier material, preferably a semi-permeable polymer, in particular, ethylcellulose; and

the majority of the granules, preferably more than 80%, more preferably more than 90%, of the granules being essentially spherical as defined by an *aspect ratio* (defined as the ratio of the length divided by the breadth) within 1.00-1.25, preferably within 1.00-1.20, more preferably within 1.00-1.15; and

the majority of the granules, preferably more than 70%, more preferably more than 90%, of the granules of the composition exerting sieve values in the range of $\geq 0.5$ mm and $<1.4$ mm, preferably in the range of $\geq 0.7$ mm and $\leq 1.1$ mm; and

the composition exerting the following in vitro dissolution rates [when measured in a model system using simulated intestinal fluid in USP Paddle System 2 operated at 37 °C with stirring speed 100 rpm]:

a) within 2-20%, preferably within 5-15 %, of the total 5-ASA is released after 15 minutes in the model system;

b) within 20-50%, preferably within 25-45%, of the total 5-ASA is released after 60 minutes in the model system;

c) within 30-70%, preferably within 40-60% of the total 5-ASA is released after 90 minutes in the model system;

d) within 50-90%, preferably within 55-80%, of the total 5-ASA is released after 150 minutes in the model system;

e) within 75-100% of the total 5-ASA is released after 240 minutes in the model system.

In the present context "5-ASA" is used as also encompassing pharmaceutically acceptable salts and esters thereof.

The salts of 5-ASA may be acid addition salts, in particular the hydrochloride, but any pharmaceutically acceptable, non-toxic organic or inorganic acid may be used.

Also salts formed with the carboxylic acid group may be used. As examples may be mentioned alkali metal salts (K, Na), alkaline earth metal salts (Ca, Mg), but again any pharmaceutically acceptable, non-toxic salt may be used. The Na- and Ca-salts are preferred.

Applicable esters are e.g.

straight chain or branched $C_1$-$C_{18}$ alkyl esters, e.g. methyl, ethyl, propyl, isopropyl, butyl, isobutyl, amyl, hexyl, heptyl, octyl, nonyl, decyl, lauryl, myristyl, cetyl, and stearyl, etc.,

straight chain or branched $C_2$-$C_{18}$ alkenyl esters, e.g. vinyl, allyl, undecenyl, oleyl, linolenyl, etc.,

$C_3$-$C_8$ cycloalkyl esters, e.g. cyclopropyl, cyclobutyl, cyclopentyl, cyclohexyl, cycloheptyl and cyclooctyl, etc.,

aryl esters, e.g. phenyl, toluyl, xylyl, naphthyl, etc.,

alicyclic esters, e.g. menthyl, etc., or

aralkyl esters, e.g. benzyl, phenethyl, etc.

## DETAILED DESCRIPTION OF THE INVENTION

The present invention provides for an oral composition in the form of granules designed for direct oral administration, i.e. the granules satisfy the pharmaceutical requirements without being formulated e.g. as tablets or e.g. formulated in capsules.

Specific problems had to be overcome, first of all the individual granules should be able to pass relatively quickly through the ventricle without any significant dissolution of 5-ASA, and subsequently a fraction of the 5-ASA should be distributed both in the small and large intestine and reside there in sufficient time for exerting the localized effect.

The present invention provides a modified release oral composition for the treatment of inflammatory bowel diseases, said composition ensuring bio-availability of 5-aminosalicylic acid (5-ASA) in both the small and large intestine, and consisting of:

individually coated granules, each granule comprising:

- a core consisting essentially of 5-aminosalicylic acid (5-ASA) or a pharmaceutically acceptable salt or ester thereof and a physiologically acceptable spheronization aid, preferably a cellulose derivative, in particular microcrystalline cellulose, and

- a coating confining said core, said coating comprising a rate-limiting barrier material, which is a diffusion-rate limiting barrier material or an erodable, degradable rate-limiting barrier material, preferably a semi-permeable polymer, in particular ethylcellulose; and

the majority of the granules, preferably more than 80%, more preferably more than 90%, of the granules being essentially spherical as defined by an *aspect ratio* (defined as the ratio of the length divided by the breadth) within 1.0-1.25, preferably within 1.00-1.20, more preferably within 1.00-1.15; and

the majority of the granules, preferably more than 70%, more preferably more than 90%, of the granules of the composition exerting sieve values in the range of ≥0.5 mm and <1.4 mm, preferably in the range of≥0.7 mm and ≤1.1 mm; and

the composition exerting the following in vitro dissolution rates [when measured in a model system using simulated intestinal fluid in USP Paddle System 2 operated at 37 °C with stirring speed 100 rpm]:

a) within 2-20%, preferably within 5-15 %, of the total 5-ASA is released after 15 minutes in the model system;

b) within 20-50%, preferably within 25-45%, of the total 5-ASA is released after 60 minutes in the model system;

c) within 30-70%, preferably within 40-60% of the total 5-ASA is released after 90 minutes in the model system;

d) within 50-90%, preferably within 55-80%, of the total 5-ASA is released after 150 minutes in the model system;

e) within 75-100% of the total 5-ASA is released after 240 minutes in the model system.

As stated, the "spheronization aid" is preferably microcrystalline cellulose. The "rate-limiting barrier material" is a coating material which acts as a diffusion-rate limiting barrier or as erodable, degradable rate-limiting barrier. The preferred material is ethylcellulose.

The composition of the invention exerts the following in vivo 5-ASA release parameters:
provided the gastric emptying is within the normal range, 50% of the granules have left the stomach within 60 minutes after intake of the composition, preferably within 30 minutes.

Furthermore, the composition exerts the following in vivo 5-ASA release parameters:
provided the small bowel transit time is within the normal range, 50% of the granules is present in the small bowel 3-6 hours after intake of the composition.

Furthermore, the composition exerts the following in vivo 5-ASA release parameters:
provided the large bowel transit time is within the normal range, 50% of the granules is present in the large bowel 12-50 hours after intake of the composition.

The transit time of various pharmaceutical formulations has been the subject of numerous studies.

Bechgaard, H., Acta Pharmaceutica Technologica 28(2), 1982, studied the critical factors influencing gastrointestinal absorption and focused on the gastrointestinal transit time and pH. She pointed to the marked difference in transit time between single-unit dosages, i.e. oral pharmaceutical formulations consisting of one non-disintegrating unit and multiple-unit dosage, i.e. oral pharmaceutical formulations consisting of a unit which disintegrates in the stomach into a large number of sub-units.

For single-unit dosage forms Bechgaard reports gastric emptying in the range from 0 to 24 hours, while the most recent studies cited (Bogentoft et al.) for multiple-unit dosage forms varies from 1.5 to 2.5 hours in fasting condition to 2.3 to 3 hours in non-fasting condition.

Bechgaard does not report overall intestinal transit time but only the transit time from mouth to caecum. Again there is a very considerable variation for single-unit dosages ranging from 5 to 40 hours, while the transit time for multiple units lie within a more narrow range. Bechgaard obtained results showing a large variation as a function of the density of the pellets, which, however, could not be verified by Bogentoft (6,1 $\pm$ 0,9 to 7,1 $\pm$ 0,8 hours).

The Pentasa ® formulation according to the above-mentioned US patents is a multiple unit formulation and the release of 5-ASA from Pentasa during normal and accelerated intestinal transit time in 7 healthy volunteers has been investigated by Christensen, L.A. et al., Br, J. Clin. Pharmac. (1987), 23, 365-369.

Daily dose was 1500 Pentasa, normal transit time (NTT) was 24 h (16-26 h) and accelerated transit time (ATT), caused by a laxative, was 5 h (4-9 h). Median total recovery (24 h, 5-ASA + acetyl-5-ASA) was 87% (61-129%) (NTT) and 81% (56-100%) (ATT), respectively, (P > 0.10). An almost complete release of 5-ASA from Pentasa takes place during NTT. At ATT conditions about 88% is released, indicating Pentasa to be an acceptable source of 5-ASA also in diarrhoeal states.

While only a relatively small group of volunteers were investigated 6 of the volunteers had NTT's in the range from 24 to 26 hours and 1 had an NTT of 16 hours.

The pH-profile and regional transit times of the normal gut has been measured by a radiotelemetry device by Fällingborg, J., et al., Aliment. Pharmacol. therap. (1989) 3, 605-613. The pH of the gut lumen was measured in 39 healthy persons using a pH-sensitive, radiotransmitting capsule. Thirteen persons were studied twice. The location of the capsule was determined by X-ray. The pH rose from 6.4 in the duodenum to 7.3 in the distal part of the small intestine. In 17 persons the pH dropped by 0.1-0.8 pH units during the last hours of the small intestinal transit. The pH was 5.7 in the caecum, but rose to 6.6 in the rectum. Gastric residence time was 1.1 h, small intestinal transit was 8 h, and colonic transit time was 17.5 h (median values). The results provide a firmer basis for prediction of the level, and the rate of release of active substance from pH-dependent sustained-release oral preparations and confirm the data obtained by Christensen op.cit.

Further advantages of the composition of the invention are related to improvements with respect to compliance and reproducibility of pharmaceutical characteristics, including laboratory characteristics, especially reproducibility of coating technique parameters.

DESCRIPTION OF THE MANUFACTURING PROCESS

5-ASA and the spheronization aid are weighed out in the predetermined ratio, e.g. wherein the % by weight of 5-ASA of the total weight of said granule ranging from 30-90%, preferably from 40-80%, more preferably from 50-60%, most preferably about 50%.

The ingredients are thoroughly mixed in a mixing container.

The next step is a granulating process comprising mixing the ingredients with a granulating agent, preferably water, e.g. in the range of 70-90% by weight of the water of the total amount of 5-ASA and other ingredients. Preferably the granulation is carried out in the mixing container.

An advantage of this step of the process is that it may be performed with water, thus avoiding using organic solvents.

In a subsequent step, an extrusion may be performed by extruding the above-mentioned mixture through sieves with pores of a diameter of e.g. 1.0 mm.

The subsequent step involves spheronization of the mixture by applying the mixture on a spheronizing apparatus, preferably a NICA spheronizer. The process is carefully monitored, and the speed and the employed time interval adjusted according to the instructions of the apparatus, e.g. operated at the maximally allowed speed, and so as to obtain the size and shape of the 5-ASA granules as specified herein.

After the spheronizing step, the granules are transferred to a fluid-bed drying system, and after drying, the granules are individually coated with the rate-limiting barrier material, preferably ethylcellulose, said material being dissolved in e.g. an organic solvent, preferably acetone, in particular in a concentration of from 0.1 - 5% w/w.

The monitoring of the obtainment of granules having the specified shapes and sizes may be performed by the following procedure:

I. Image processing and analysis:
A commercially available microscope and analysis software was obtained from Leica ("Leica Q500MC Image Analysis System") and was used to determine the dimensions and the aspect ratio of the prepared granules.
The aspect ratio as used herein is defined as the ratio of the length divided by the breadth. The length is defined as the length of the longest dimension of the granule. The breadth is defined as the length of the shortest dimension of the granule.

Samples were taken up randomly, e.g. in triplicate. The compositions according to the invention should meet the following criteria:

the majority of the granules, preferably more than 80%, more preferably more than 90%, of the granules are essentially spherical as defined by an *aspect ratio* within 1.00-1.25, preferably within 1.00-1.20, more preferably within 1.00-1.15.

II. Furthermore, the particle size distribution of the granules of the composition can be determined by the LEICA ANALYSIS SYSTEM as described above, and also in the following way:

Representative samples of a granule preparation are sieved over a sieve-stack of varying sieves, using fixed time and oscillation, the sieves typically being: 1.40 mm - 1.25 mm - 1.12 mm - 1.00 mm - 0.710 mm - 0.50 mm - 0.355 mm - 0.250 mm.

The compositions according to the invention should meet the following criteria:

the majority of the granules, preferably more than 70%, more preferably more than 90%, of the granules of the composition exerting sieve values in the range of ≥0.5 mm and <1.4 mm, preferably in the range of ≥0.7 mm and ≤1.1 mm.

Furthermore, the prepared granule preparations are tested in an in vitro model system for dissolution profiles and using simulated intestinal fluid, 0.1 M Na phosphate buffer, pH 7.5, in USP Paddle System 2 operated at 37 °C with stirring speed 100 rpm. Batches exerting the dissolution profiles described below are selected for clinical purposes. The preferred dissolution profiles of the granules of the invention are as follows:

a) within 2-20%, preferably within 5-15 %, of the total 5-ASA is released after 15 minutes in the model system;

b) within 20-50%, preferably within 25-45%, of the total 5-ASA is released after 60 minutes in the model system;

c) within 30-70%, preferably within 40-60% of the total 5-ASA is released after 90 minutes in the model system;

d) within 50-90%, preferably within 55-80%, of the total 5-ASA is released after 150 minutes in the model system;

e) within 75-100 % of the total 5-ASA is released after 240 minutes in the model system.

DETAILED DESCRIPTION OF THE GEOMETRICAL/ STRUCTURAL CHARACTERISTICS OF THE GRANULES

Granules of the invention are selected so as to exert the following geometrical/structural characteristics:

the majority of the granules, preferably more than 80%, more preferably more than 90%, of the granules being essentially spherical as defined by an *aspect ratio* within 1.00-1.25, preferably within 1.00-1.20, more preferably within 1.00-1.15; and

the majority of the granules, preferably more than 70%, more preferably more than 90%, of the granules of the composition exerting sieve values in the range of ≥0.5 mm and <1.4 mm, preferably in the range of ≥0.7 mm and ≤1.1 mm.

**PREFERRED EMBODIMENT OF THE INVENTION**

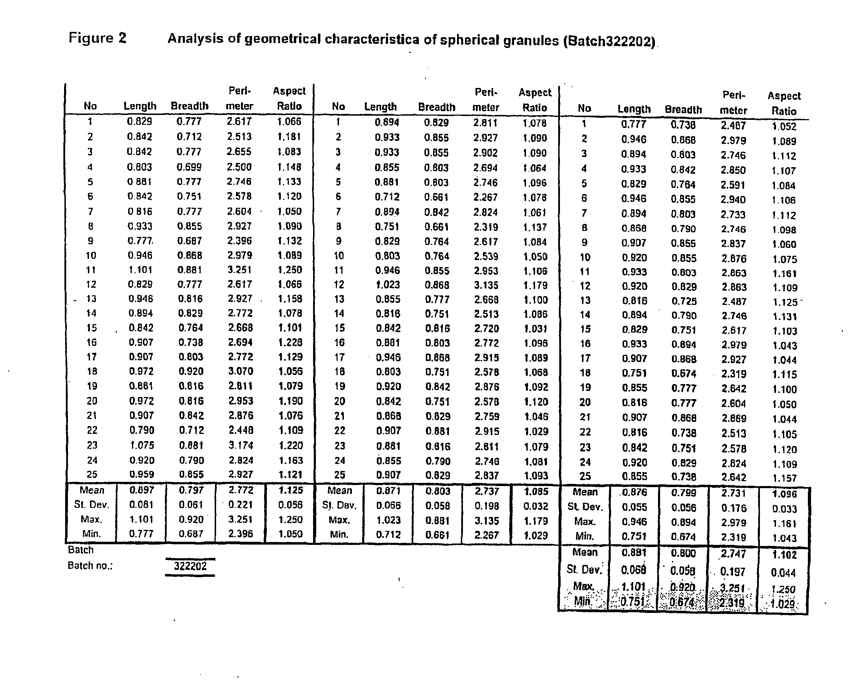

**(BATCH 322202) - see Figure 2**

For preferred granules according to the invention, the following results were obtained (measurements based on 75 measurements on granules obtained by random sampling)

LENGTH

With respect to **the length** of the granule as defined herein as the length of the longest dimension of the granule, the following values were obtained:

Minimum = 0.751 mm - maximum = 1.101 mm,

i.e. the range was from 0.75 to 1.10 mm.

The granules showed length values varying within (mean ± 1 SD): 0.881 mm ± 0.068 mm = from 0.813 mm to 0.949 mm - and the granules had maximal length varying within mean ± 2 SD: 0.881 mm ± 0.136 mm = from 0.745 mm to 1.017 mm. Thus, the majority (95%) of the granules showed a **length** (the length of the longest dimension) of from **0.75 mm to 1.02 mm.**

BREADTH

With respect to **the breadth** of the granules defined herein as the length of the shortest dimension of the granule, the following values were obtained:

Minimum = 0.674 mm - maximum = 0.920 mm,

i.e. the range was from 0.67 to 0.92 mm.

The granules had breadth values varying within (mean ± 1 SD): 0.800 mm ± 0.058 mm = from 0.742 mm to 0.858 mm - and within (mean ± 2 SD): 0.800 mm ± 0.116 mm = from 0.684 mm to 0.916 mm. As seen, the majority. 95%) of the granules showed

**breadth** (the length of the shortest dimension) of from **0.68 mm to 0.92 mm.**

ASPECT RATIO

With respect to the **aspect ratio** defined herein as the ratio of the length divided by the breadth, (the length being defined as the length of the longest dimension of the granule, and the breadth being defined as the length of the shortest dimension of the granule), the following values were obtained:

$$\text{Minimum} = 1.029 - \text{maximum} = 1.250$$

i.e. the range of aspects ratios was from 1.03 to 1.25.
The granules showed aspect ratios varying within (mean ± 1 SD): 1.102 ± 0.044 = from 1.058 to 1.146 - and within (mean ± 2 SD):= 1.102 ± 0.088 mm = from 1.014 to 1.190. As seen, the majority, 97 %) of the granules showed

**aspect ratios** of from **1.01 to 1.19.**

**PREFERRED EMBODIMENT OF THE INVENTION**

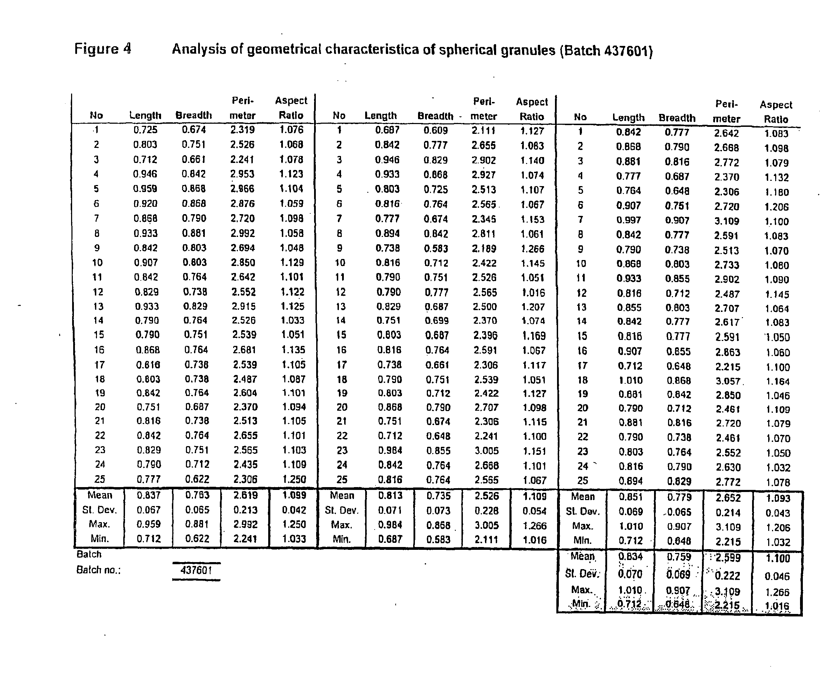

**(BATCH 437601) - see Figure 4**

For preferred granules according to the invention, the following results were obtained (measurements based on 75 measurements on granules obtained by random sampling)

LENGTH

With respect to **the length** of the granule as defined herein as the length of **the** longest dimension of the granule, the following values were obtained:

$$\text{Minimum} = 0.712 \text{ mm} - \text{maximum} = 1.010 \text{ mm,}$$

i.e. the range was from 0.71 to 1.01 mm.
The granules had length values varying within (mean ± 1 SD): 0.834 mm ± 0.070 mm = from 0.764 mm to 0.904 mm; and within (mean ± 2 SD): 0.834 mm ± 0.140 mm = from 0.694 mm to 0.974 mm. As seen, the majority, 98 %) of the granules showed a **length** (the length of longest dimension) of from **0.69 mm to 1.02 mm.**

BREADTH

With respect to **the breadth** of the granules defined herein as the length of the shortest dimension of the granule, the following values were obtained:

$$\text{Minimum} = 0.648 \text{ mm} - \text{maximum} = 0.907 \text{ mm,}$$

i.e. the range was from 0.65 to 0.91 mm.
The granules had breadth values varying within (mean ± 1 SD):= 0.759 mm ± 0.069 mm = from 0.690 mm to 0.828 mm; and within (mean ± 2 SD): 0.759 mm ± 0.138 mm = from 0.621 mm to 0.897 mm. As seen, the majority 95% of the granules showed **breadth** (the length of the shortest dimension) of from **0.62 mm to 0.90 mm.**

ASPECT RATIO

With respect to the **aspect ratio** defined herein as the ratio of the length divided by the breadth, (the length being defined as the length of the longest dimension of the granule, and the breadth being defined as the length of the shortest dimension of the granule), the following values were obtained:

$$\text{Minimum} = 1.016 - \text{maximum} = 1.266$$

i.e. the range of aspects ratios was from 1.02 to 1.27.
The granules showed aspect ratios varying within (mean + 1 SD): 1.100 ± 0.046 = from 1.054 to 1.146; and within (mean ± 2 SD): 1.100 ± 0.092 mm = from 1.008 to 1.192. As seen, the majority (approx. 95%) of the granules showed **aspect ratios** of from **1.01 to 1.19.**

TREATMENT OF INFLAMMATORY BOWEL DISEASES

A main aspect of the invention is a method for the treatment of inflammatory bowel diseases (IBD) in particular Crohn's disease, colitis ulcerosa, an unclassified form of said diseases, or a diagnosed subtype of said disease comprising orally administering a pharmacologically effective amount of the composition according to the invention.
The term "pharmacologically effective amount" as used herein, represents an amount of a compound of the invention which is capable of inducing the desired therapeutic effect in the individual in need thereof. The particular dose of 5-ASA administered according to the present invention will, of course, be determined by the particular circumstances relating to the case, including the particular condition and pathological site to be treated, the sex, age, and weight of the individual, and similar considerations.

The present invention is also useful in a maintenance treatment of more or less chronic inflammatory bowel disease, inter alia because systemic effects and other adverse effects due to the 5-ASA are negligible. Thus, relatively long treatment cycles employing relatively high total amounts of drugs may be prescribed with the concomitant reduced risk of adverse effects.

The target part of the gastrointestinal tract is a target part in the proximal small intestine, the mid small intestine, the distal small intestine, the caecum, the ascending colon, the transverse colon, the descending colon, the sigmoid colon and/or the rectum.

An aspect of the invention is a composition, wherein the 5-ASA is in a unit dosage form and comprises 5-ASA in amounts suitable for the administration of from 250 mg to 12 g, preferably from 500 mg to 6 g, more preferably from 500 mg to 4 g, e.g. in unit dosage form each comprising 500 mg, 1 g, 2 g, 5 g, or 6 g.

The term "unit dosage form" refers to physically discrete units suitable as unitary dosages for clinical use, each unit containing a predetermined quantity of 5-ASA calculated to produce the desired therapeutic effect.

Furthermore, the composition is preferably a composition, wherein the 5-ASA is supplied as a unit dosage forms in sealed packages to be opened immediately prior to use, e.g. sachets or sticks.

## BRIEF DESCRIPTION OF THE DRAWINGS

FIG. 1 shows an image analysis of spherical granules of a preferred embodiment of the invention prepared as described below.

FIG. 2 shows the corresponding *aspect ratio* determinations, obtained by LEICA Q500MC Image Analysis System.

FIG. 3 and 4 show similar data obtained from another batch.

FIG. 5 is a graphical depiction of the preferred dissolution rate intervals of the granules according to the invention.

FIG. 6 is a graphical depiction showing the same dissolution rate intervals as in FIG. 5, but also showing data obtained from a comparison experiment:
It is seen that the dissolution profile of the conventional granules are very different from the profiles of the granules of the invention.

FIG. 7 is a graphical depiction of data obtained from spherical granules of the invention. The test procedure was as in FIG. 6. Results are within the preferred limits.

FIG. 8 is a graphical depiction of data obtained from spherical granules of the invention. The test procedure was as in FIG. 6. Results are within the preferred limits.

FIG. 9 is a graphical depiction showing the results described in FIG. 6 and 7 and 8 on the same graph.

FIG. 10 shows a table relating to in vivo gastric emptying time and colon arrival time of spherical granules.

FIG. 11 and FIG. 12 show results from the same clinical study as described in FIG: 10: plasma concentration curves of 5-ASA.

## EXAMPLE 1

THE MANUFACTURE OF GRANULE COMPOSITIONS ACCORDING TO THE INVENTION

5000 g 5-ASA and 5000 g microcrystalline cellulose were weighed out and carefully mixed at a fixed time and speed. 8000 g purified water was poured into the blending container and the ingredients were mixed.

The mixture was extruded through 1.0 mm sieves, and spheronized at fixed time and speed. After carefully monitored spheronization, using maximal speed (790 rpm) of the NICA spheronizing apparatus (NICA S2-450) for a fixed interval of time, e.g. 5 minutes, being adapted to the amount of granule mixture applied to the apparatus, the spheronized granules were transferred to a fluid-bed drying system. After drying, the pellets were spray-coated with ethyl cellulose dissolved in acetone.

Finally, the granules were analyzed and selected as described above, with respect to the geometrical properties.

For determination of the particle size distribution, typically 400 grams of granules were used. The determination was carried out with a Retsch laboratory sieving machine type VIBRO (1.400 - 1.250 - 1.120 - 1.000 - 0.710 - 0.500 - 0.355 - 0.250 mm). The oscillation amplitude control regulation was set at 60, for 10 minutes.

The specific batches of granules prepared as described above are further characterized by the accompanying drawings.

DESCRIPTION OF THE TESTS AND RESULTS

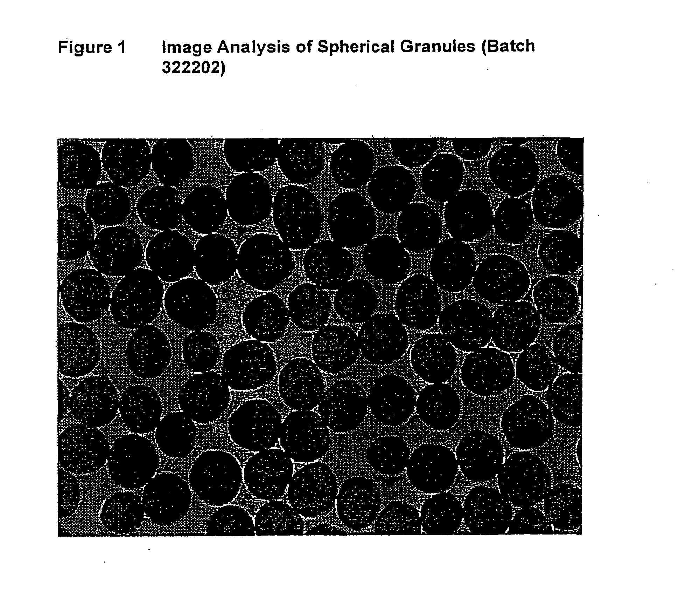

An image analysis of spherical granules of a preferred embodiment (batch 322201) of the invention is shown in FIG. 1.

FIG. 2 is a table showing corresponding geometrical characteristics obtained from the same batch., including data representing length, breadth, and *aspect*

*ratio* determinations - all obtained by the LEICA Q500MC Image Analysis System. Typically approximately 3 x 300 mg of granules are taken randomly for image processing and analysis.

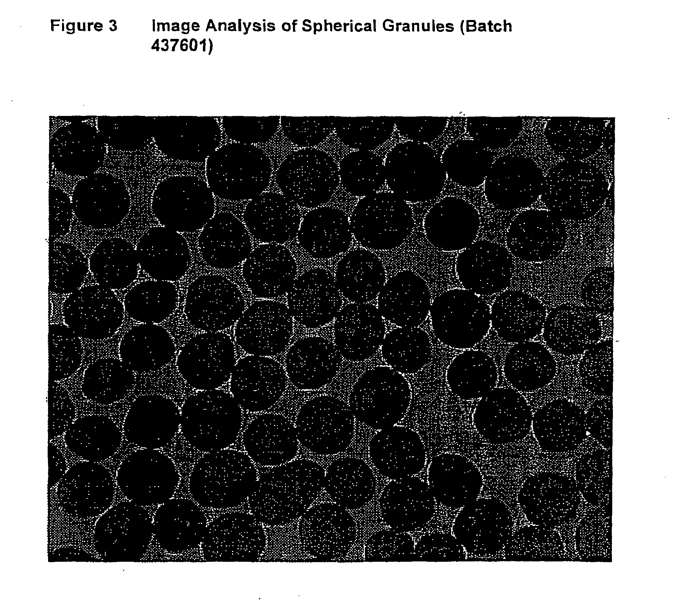

FIG. 3 and 4 show image analysis and geometrical data obtained from batch 437601.

Test procedure for dissolution rates:

The in vitro dissolution rates were tested in simulated intestinal fluid using a USP Paddle system 2 Dissolution System. The following conditions were applied:

Dissolution Fluid: 0.1 M Na-phosphate buffer pH = 7.5

Fluid Volume: 1000 ml

Temperature: 37 °C

Stirring speed: 100 rpm

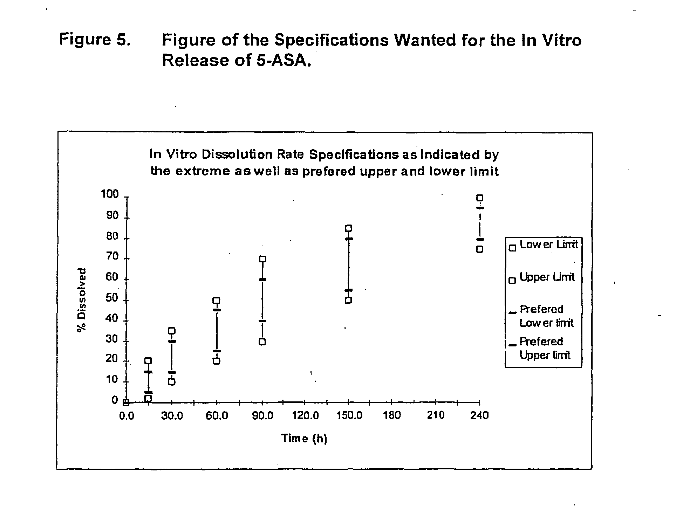

A graphical depiction of the preferred dissolution rate intervals of the granules according to the invention is shown in fig. 5.

COMPARISON STUDY

A comparison study was performed using a portion of some granules representing a 5-ASA granulate prepared as an intermediary product prior to the addition of tablet ingredients and compression into tablets, i.e. an intermediary 5-ASA granulate as prepared prior to the formulation of conventional 5-ASA tablets. The comparison results showed that such "conventional" granules did not provide the properties characteristic of the granules of the present invention. The conventional granules employed in the comparison study were granules made from a homogenous mixture of 5% polyvinylpyrrolidone and 95% 5-ASA, and granulated and extruded (1.0 mm sieve), and subsequently coated with ethylcellulose.

6 batches were tested.

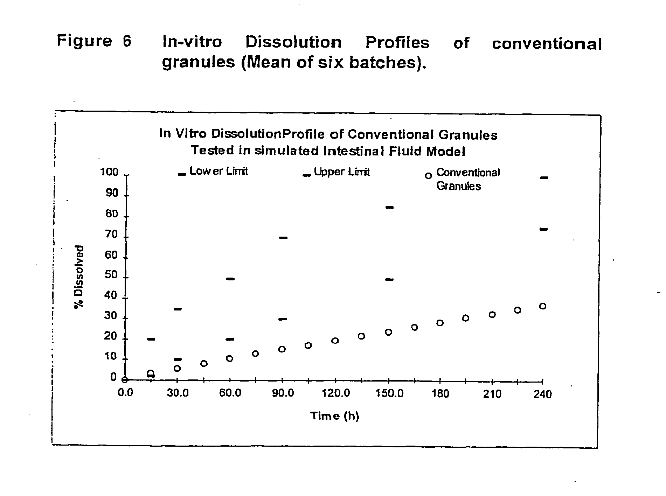

FIG. 6 is a graphical depiction showing the same dissolution rate intervals as in FIG. 5, but also showing data obtained from a comparison experiment: It is seen that the dissolution profile of the conventional granules is very different from the profiles of the granules of the invention.

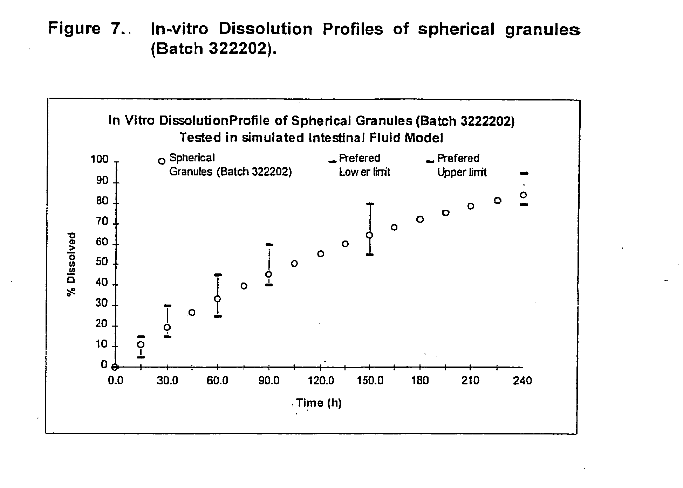

FIG. 7 is a graphical depiction of data obtained from spherical granules of the invention (batch 322202). The test procedure was as in FIG. 6. Results are within the preferred limits.

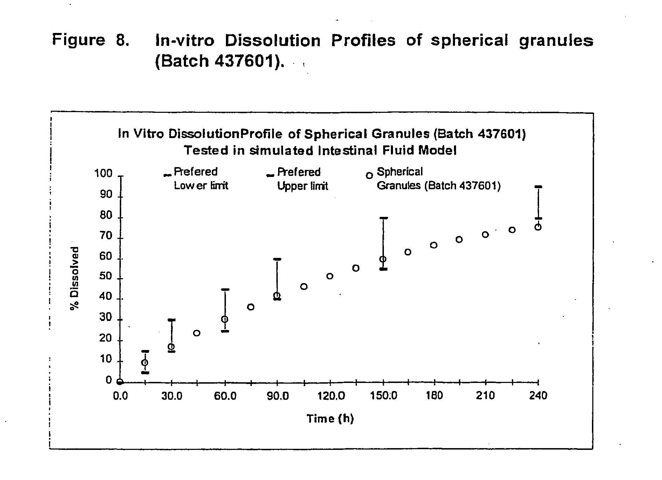

FIG. 8 is a graphical depiction of data obtained from spherical granules of the invention (batch 437601). The test procedure was as in FIG. 6. Results are within the preferred limits.

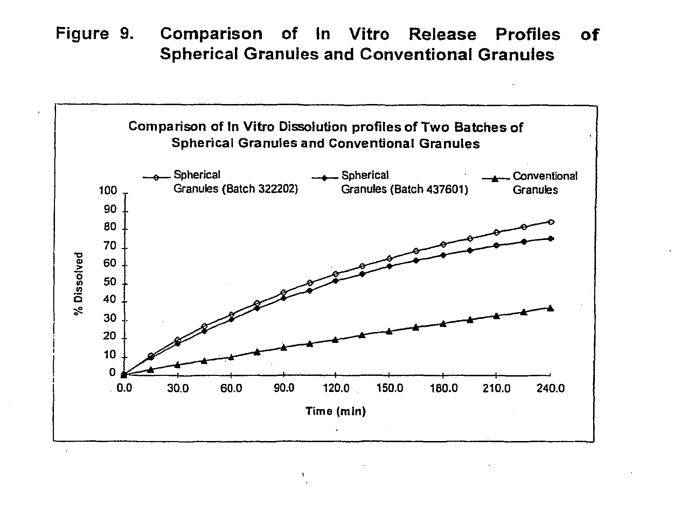

FIG. 9 is a graphical depiction showing the results described in FIG. 6 (comparison data) and FIG. 7 and FIG: 8 on the same graph.

Test procedure in vivo

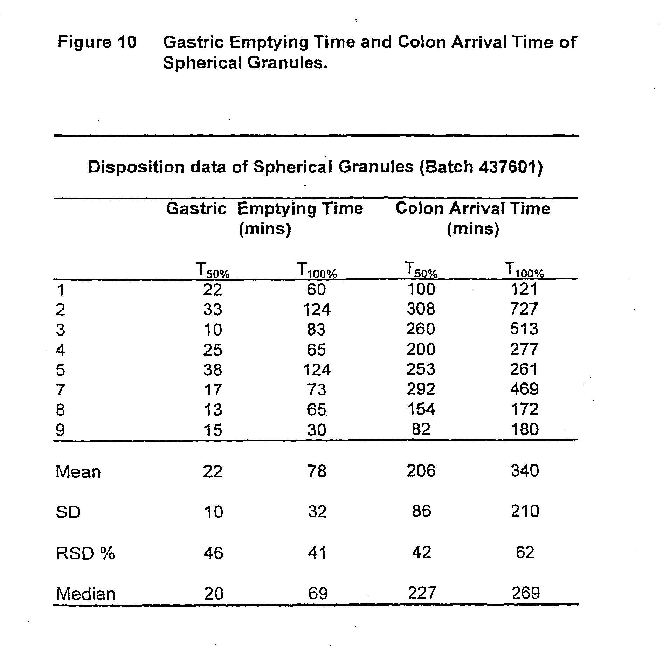

The dispositions of spherical granules were investigated in eight healthy volunteers. The experiments were performed according to a Clinical Study Protocol employed at *Pharmaceutical Profiles Ltd,* Nottingham, UK., using radiolabelled $^{153}$Sm granules for the localization of the position of the disposed composition. The results are shown in FIG. 10, which is a table showing the gastric emptying time and colon arrival time of the tested spherical granules.

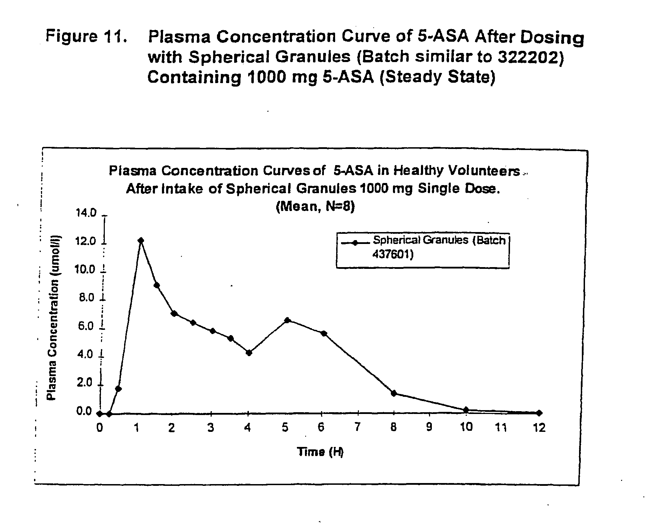

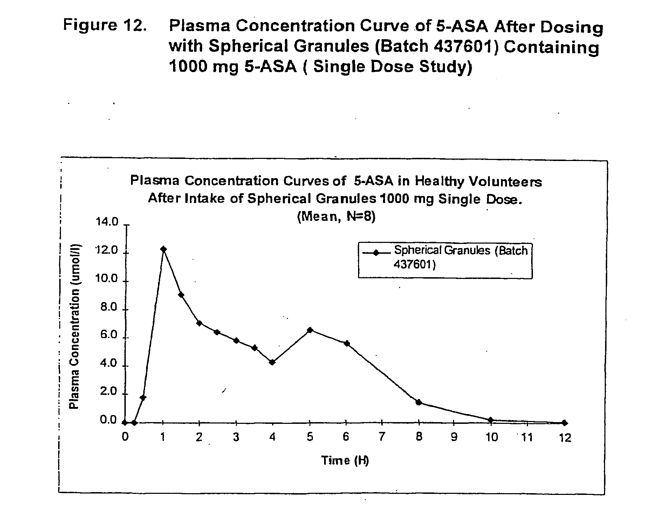

FIG. 11 and FIG. 12 show further results from the clinical study: plasma concentration curves of 5-ASA after administration (1000 mg single dose) of the granule composition of the invention.

**Claims**

1. A modified release oral composition for the treatment of inflammatory bowel diseases, said composition ensuring bioavailability of 5-aminosalicylic acid (5-ASA) in both the small and large intestine, and consisting of

individually coated granules, each granule comprising:

- a core consisting essentially of 5-aminosalicylic acid (5-ASA) or a pharmaceutically acceptable salt or ester thereof and a physiologically acceptable spheronization aid, preferably a cellulose derivative, in particular microcrystalline cellulose, and
- a coating confining said core, said coating comprising a rate-limiting barrier-material, which is a diffusion-rate limiting barrier material or an erodable, degradable rate-limiting barrier material, preferably a semi-permeable polymer, in particular ethylcellulose; and

the majority of the granules, preferably more than 80%, more preferably more than 90%, of the granules being essentially spherical as defined by an *aspect ratio* (defined as the ratio of the length divided by the breadth) within 1.00-1.25, preferably within 1.00-1.20, more preferably within 1.00-1.15; and
the majority of the granules, preferably more than 70%, more preferably more than 90%, of the individual granules of the composition exerting sieve values in the range of ≥0.5 mm and <1.4 mm, preferably in the range of ≥0.7 mm and ≤1.1; and
the composition exerting the following in vitro dissolution rates [when measured in a model system using simulated intestinal fluid in USP Paddle System 2 operated at 37 °C with stirring speed 100 rpm]:

> a) within 2-20%, preferably within 5-15 %, of the total 5-ASA is released after 15 minutes in the model system;
> b) within 20-50%, preferably within 25-45%, of the total 5-ASA is released after 60 minutes in the model system;
> c) within 30-70%, preferably within 40-60% of the total 5-ASA is released after 90 minutes in the model system;
> d) within 50-90%, preferably within 55-80%, of the total 5-ASA is released after 150 minutes in the model system;
> e) within 75-100% of the total 5-ASA is released after 240 minutes in the model system.

2. A composition according to claim 1 exerting the following in vivo 5-ASA release parameters:
provided the gastric emptying is within the normal range, 50% of the granules have left the stomach within 60 minutes after intake of the composition, preferably within 30 minutes.

3. A composition according to claim 1 exerting the following in vivo 5-ASA release parameters:
provided the small bowel transit time is within the normal range, 50% of the granules is present in the small bowel 3-6 hours after intake of the composition.

4. A composition according to claim 1 exerting the following in vivo 5-ASA release parameters:
provided the large bowel transit time is within the normal range, 50% of the granules is present in the large bowel 12-50 hours after intake of the composition.

5. A composition according to claim 1, wherein the % by weight of 5-ASA of the total weight of each granule ranging from 30-90%, preferably from 40-80%, more preferably from 50-60%.

6. A composition according to claim 1, wherein the 5-ASA is in a unit dosage form and comprises 5-ASA in amounts suitable for the administration of from 250 mg to 12 g, preferably from 500 mg to 6 g, more preferably from 500 mg to 4 g, e.g. in unit dosage form each comprising 500 mg, 1 g, 2 g, 5 g, or 6 g.

7. A composition according to claim 1, wherein the 5-ASA is supplied as unit dosage forms in sealed packages to be opened immediately prior to use, e.g. sachets or sticks.

8. A composition according to claim 1, wherein the inflammatory bowel disease is Crohn's disease, colitis ulcerosa, an unclassified form of said diseases, or a diagnosed subtype of said disease.

9. Use of 5-ASA or a pharmaceutically acceptable salt or ester thereof for the manufacture of a medicament for the treatment of inflammatory bowel diseases (IBD), in particular Crohn's disease, colitis ulcerosa, an unclassified form of said diseases, or a diagnosed subtype of said disease, said medicament consisting of

> individually coated granules, each granule comprising:
>
> > - a core consisting essentially of 5-aminosalicylic acid (5-ASA) (or a salt or an ester thereof) and a physiologically acceptable spheronization aid, preferably a cellulose derivative, in particular microcrystalline cellulose, and
> > - a coating confining said core, said coating comprising a rate-limiting barrier-material, which is a diffusion-rate limiting barrier material or an erodable, degradable rate-limiting barrier material, preferably a semi-permeable polymer, in particular ethylcellulose; and
>
> the majority of the granules, preferably more than 80%, more preferably more than 90%, of the granules being essentially spherical as defined by an *aspect ratio* (defined as ratio of the length divided by the breadth) within 1.00-1.25, preferably within 1.00-1.20, more preferably within 1.00-1.15; and
> the majority of the granules, preferably more than 70%, more preferably more than 90%, of the individual granules of the composition exerting sieve values in the range of ≥0.5 mm and <1.4 mm, preferably in the range of ≥0.7 mm and ≤1.1; and
> the composition exerting the following in vitro dissolution rates [when measured in a model system using simulated intestinal fluid in USP Paddle System 2 operated at 37 °C with stirring speed 100 rpm]:
>
> > a) within 2-20%, preferably within 5-15 %, of the total 5-ASA is released after 15 minutes in the model system;
> > b) within 20-50%, preferably within 25-45%, of the total 5-ASA is released after 60 minutes in the model system;
> > c) within 30-70%, preferably within 40-60% of the total 5-ASA is released after 90 minutes in the model system;

d) within 50-90%, preferably within 55-80%, of the total 5-ASA is released after 150 minutes in the model system;

e) within 75-100% of the total 5-ASA is released after 240 minutes in the model system.

**Patentansprüche**

1. Orale Zusammensetzung mit modifizierter Freisetzung zur Behandlung von entzündlichen Darmerkrankungen, wobei die Zusammensetzung eine Bioverfügbarkeit von 5-Aminosalicylsäure (5-ASA) im Dick- und Dünndarm sicherstellt und aus einzeln überzogenen Kügelchen besteht, wobei jedes Kügelchen Folgendes umfasst:

einen im Wesentlichen aus 5-Aminosalicylsäure (5-ASA) oder einem pharmazeutisch zulässigen Salz oder einem Ester desselben und einem physiologisch zulässigen Spheronizing-Hilfsmittel, vorzugsweise einem Cellulosederivat, insbesondere mikrokristalliner Cellulose; bestehenden Kern

- einen Überzug, der den Kern umhüllt, wobei der Überzug ein ratenbegrenzendes Ummantelungsmaterial umfasst, das ein diffusionsratenbegrenzendes Ummantelungsmaterial oder ein erodierbares, abbaubares ratenbegrenzendes Ummantelungsmaterial ist, vorzugsweise ein halbpermeables Polymer, insbesondere Ethylcellulose, und

die Mehrheit der Kügelchen, vorzugsweise mehr als 80%, noch bevorzugter mehr als 90% der Kügelchen im Wesentlichen kugelförmig sind, was durch ein *Aspektverhältnis* (definiert als das Verhältnis der Länge dividiert durch die Breite) innerhalb von 1,00 -1,25, vorzugsweise innerhalb von 1,00 - 1,20, noch bevorzugter innerhalb von 1,00 - 1,15 definiert wird, und

die Mehrheit der Kügelchen, vorzugsweise mehr als 70%, noch bevorzugter mehr als 90% der einzelnen Kügelchen der Zusammensetzung Siebwerte in dem Bereich von ≥ 0,5 mm und < 1,4 mm, vorzugsweise in dem Bereich von ≥ 0,7 mm und ≤ 1,1 mm ausüben, und

die Zusammensetzung die folgenden in vitro Auflösungsraten ausübt [bei Messung in einem Modellsystem unter Verwendung einer simulierten Darmflüssigkeit im USP Paddle System 2 betrieben bei 37°C mit einer Rührgeschwindigkeit von 100 U/min.]:

a) innerhalb 2 - 20%, vorzugsweise innerhalb 5-15% des gesamten 5-ASA wird nach 15 Minuten in dem Modellsystem freigegeben;

b) innerhalb 20 - 50%, vorzugsweise innerhalb 25 - 45% des gesamten 5-ASA wird nach 60 Minuten in dem Modellsystem freigegeben;

c) innerhalb 30 - 70%, vorzugsweise innerhalb 40 - 60% des gesamten 5-ASA wird nach 90 Minuten in dem Modellsystem freigegeben;

d) innerhalb 50 - 90%, vorzugsweise innerhalb 55 - 80% des gesamten 5-ASA wird nach 150 Minuten in dem Modellsystem freigegeben;

e) innerhalb 75 - 100% des gesamten 5-ASA wird nach 240 Minuten in dem Modellsystem freigegeben.

2. Zusammensetzung nach Anspruch 1, die die folgenden in vivo 5-ASA-Freisetzungparameter ausübt:

vorausgesetzt, die gastrische Leerung liegt innerhalb des normalen Bereichs, verlassen 50% der Kügelchen den Magen innerhalb von 60 Minuten nach Einnahme der Zusammensetzung, vorzugsweise innerhalb von 30 Minuten.

3. Zusammensetzung nach Anspruch 1, die die folgenden in vivo 5-ASA-Freisetzungparameter ausübt:

vorausgesetzt, die Dünndarm-Durchtrittszeit liegt innerhalb des normalen Bereichs, befinden sich 50% der Kügelchen 3-6 Stunden nach Einnahme der Zusammensetzung im Dünndarm.

4. Zusammensetzung nach Anspruch 1, die die folgenden in vivo 5-ASA-Freisetzungparameter ausübt:

vorausgesetzt, die Dickdarm-Durchtrittszeit liegt innerhalb des normalen Bereichs, befinden sich 50% der Kügelchen 12-50 Stunden nach Einnahme der Zusammensetzung im Dickdarm.

5. Zusammensetzung nach Anspruch 1, **dadurch gekennzeichnet, dass** der Masseprozentsatz des 5-ASA des Gesamtgewichts jedes Kügelchens von 30 - 90%, vorzugsweise von 40 - 80%, noch bevorzugter von 50 - 60% reicht.

6. Zusammensetzung nach Anspruch 1, **dadurch gekennzeichnet, dass** das 5-ASA in einer Einheitsdosierungsform vorliegt und 5-ASA in Mengen umfasst, die für die Verabreichung von 250 mg bis 12 g, vorzugsweise von 500 mg bis 6 g, bevorzugter von 500 mg bis 4 g geeignet sind, zum Beispiel in einer Einheitsdosierungsform, die jeweils 500 mg, 1 g, 2 g, 5 g oder 6 g umfasst.

7. Zusammensetzung nach Anspruch 1, **dadurch gekennzeichnet, dass** das 5-ASA als Einheitsdosierungsformen in versiegelten Packungen geliefert wird, die unmittelbar vor Gebrauch geöffnet werden, z.B. Beutelchen oder Stifte.

8. Zusammensetzung nach Anspruch 1, **dadurch gekennzeichnet, dass** die entzündliche Darmerkrankung Morbus Crohn, Colitis ulcerosa, eine nicht klassifizierte Form dieser Erkrankungen oder eine diagnostizierte Unterart dieser Erkrankung ist.

9. Verwendung von 5-ASA oder eines pharmazeutisch zulässigen Salzes oder Esters desselben zur Herstellung eines Medikaments zur Behandlung entzündlicher Darmerkrankungen, insbesondere von Morbus Crohn, Colitis ulcerosa, einer nicht klassifizierten Form dieser Erkrankungen oder einer diagnostizierten Unterart dieser Erkrankung, wobei das Medikament aus einzeln überzogenen Kügelchen besteht und jedes Kügelchen Folgendes umfasst:

    einen im Wesentlichen aus 5-Aminosalicylsäure (5-ASA) (oder einem Salz oder einem Ester desselben) und einem physiologisch zulässigen Spheronizing-Hilfsmittel, vorzugsweise einem Cellulosederivat, insbesondere mikrokristalliner Cellulose, bestehenden Kern

        - einen Überzug, der den Kern umhüllt, wobei der Überzug ein ratenbegrenzendes Ummantelungsmaterial umfasst, das ein diffusionsratenbegrenzendes Ummantelungsmaterial oder ein erodierbares, abbaubares ratenbegrenzendes Ummantelungsmaterial ist, vorzugsweise ein halbpermeables Polymer, insbesondere Ethylcellulose, und

    die Mehrheit der Kügelchen, vorzugsweise mehr als 80%, noch bevorzugter mehr als 90% der Kügelchen im Wesentlichen kugelförmig sind, was durch ein *Aspektverhältnis* (definiert als das Verhältnis der Länge dividiert durch die Breite) innerhalb von 1,00 - 1,25, vorzugsweise innerhalb von 1,00 - 1,20, noch bevorzugter innerhalb von 1,00-1,15 definiert wird, und
die Mehrheit der Kügelchen, vorzugsweise mehr als 70%, noch bevorzugter mehr als 90% der einzelnen Kügelchen der Zusammensetzung Siebwerte in dem Bereich von ≥ 0,5 mm und < 1,4 mm, vorzugsweise in dem Bereich von ≥ 0,7 mm und ≤ 1,1 mm ausüben, und
die Zusammensetzung die folgenden <u>in vitro</u> Auflösungsraten ausübt [bei Messung in einem Modellsystem unter Verwendung einer simulierten Darmflüssigkeit im USP Paddle System 2 betrieben bei 37°C mit einer Rührgeschwindigkeit von 100 U/min.]:

    a) innerhalb 2 - 20%, vorzugsweise innerhalb 5-15% des gesamten 5-ASA wird nach 15 Minuten in dem Modellsystem freigegeben;
    b) innerhalb 20 - 50%, vorzugsweise innerhalb 25 - 45% des gesamten 5-ASA wird nach 60 Minuten in dem Modellsystem freigegeben;
    c) innerhalb 30 - 70%, vorzugsweise innerhalb 40 - 60% des gesamten 5-ASA wird nach 90 Minuten in dem Modellsystem freigegeben;
    d) innerhalb 50 - 90%, vorzugsweise innerhalb 55 - 80% des gesamten 5-ASA wird nach 150 Minuten in dem Modellsystem freigegeben;
    e) innerhalb 75 - 100% des gesamten 5-ASA wird nach 240 Minuten in dem Modellsystem freigegeben.

## Revendications

1. Composition à administrer par voie orale et à libération modifiée, destinée au traitement de troubles inflammatoires intestinaux, cette composition assurant la bio-disponibilité de l'acide 5-aminosalicylique (5-ASA) à la fois dans l'intestin grêle et dans le gros intestin et comprenant :

    des granulés enrobés individuellement chaque granulé comprenant :

        - un noyau comprenant essentiellement de l'acide 5-aminosalicylique (5-ASA) ou l'un de ses sels ou esters acceptables pharmaceutiquement et un adjuvant de sphéronisation acceptable physiologiquement, de préférence un dérivé de cellulose, en particulier de la cellulose microcristalline, et,
        - - un enrobage enrobant le noyau, l'enrobage comprenant une matière formant barrière limitant la vitesse, qui est une matière formant barrière limitant la vitesse de diffusion ou une matière formant barrière pouvant s'éroder, se dégrader et limitant la vitesse, de préférence un polymère semi-perméable, en particulier de l'éthycellulose ; et

    la majorité des granulés, de préférence plus de 80 % et mieux encore plus de 90 % des granulés, étant essentiellement sphériques tels que définis par un rapport d'aspect (rapport de la longueur à la largeur) compris entre 1,00 et 1,25, de préférence entre 1,00 et 1,20 et mieux encore entre 1,00 et 1,15 ; et

la majorité des granulés, de préférence plus de 70 %, et mieux encore plus de 90 % des granulés de la composition ayant des valeurs granulométriques dans l'intervalle allant de ≥ 0,5 à < 1,4 mm, de préférence dans l'intervalle allant de ≥0,7à≤1,1 mm; et

la composition ayant les vitesses suivantes de dissolution in vitro [telles que mesurées dans un système modèle utilisant un fluide intestinal de simulation dans le système à pale N° 2 de la pharmacopée des Etats-Unis d'Amérique fonctionnant à 37°C à une vitesse d'agitation de 100 tours à la minute] :

 a) entre 2 et 20 %, de préférence entre 5 et 15 % du 5-ASA total est libéré après 15 minutes dans le système modèle ;
 b) entre 20 et 50 %, de préférence entre 25 et 45 % du 5-ASA total est libéré après 60 minutes dans le système modèle ;
 c) entre 30 et 70 %, de préférence entre 40 et 60 % du 5-ASA total est libéré après 90 minutes dans le système modèle ;
 d) entre 50 et 90 %, de préférence entre 55 et 80 % du 5-ASA total est libéré après 150 minutes dans le système modèle ;
 e) entre 75 et 100 % du 5-ASA total est libéré après 240 minutes dans le système modèle.

**2.** Composition suivant la revendication 1, ayant les paramètres de libération suivants du 5-ASA in vivo:

 pourvu que la vidange gastrique soit dans la plage normale, 50 % des granulés ont quitté l'estomac dans les 60 minutes après la prise de la composition, de préférence dans les 30 minutes.

**3.** Composition suivant la revendication 1, ayant les paramètres de libérations suivants du 5-ASA in vivo :

 pourvu que le temps de transit dans l'intestin grêle soit dans la plage normale, 50 % des granulés sont présents dans l'intestin grêle, 3 à 6 heures après la prise de la composition.

**4.** Composition suivant la revendication 1, ayant les paramètres suivants de libération du 5-ASA in-vivo :

 pourvu que le temps de transit dans le gros intestin soit dans la plage normale, 50 % des granulés sont présents dans le gros intestin 12 à 50 heures après la prise de la composition.

**5.** Composition suivant la revendication 1, dans laquelle le pourcentage en poids du 5-ASA par rapport au pourcentage total des granulés va de 30 à 90 %, de préférence de 40 à 80 % et mieux encore de 50 à 60 %.

**6.** Composition suivant la revendication 1, dans laquelle le 5-ASA est sous la forme d'unité de prise et comprend du 5-ASA en des quantités qui conviennent pour l'administration de 250 mg à 12 g., de préférence de 500 mg à 6 g. et mieux encore de 500 mg à 4 g., par exemple sous forme d'unité de prise comprenant chacune 500 mg; 1g. 2g. 5 g. ou 6 g.

**7.** Composition suivant la revendication 1, dans laquelle le 5-ASA est fourni sous forme d'unité de prise en emballage scellé à ouvrir immédiatement avant l'utilisation par exemple en sachets ou en bâtonnets.

**8.** Composition suivant la revendication 1, dans laquelle le trouble inflammatoire de l'intestin est la maladie de Crohn, la colite ulcéreuse, une forme non classée de ces maladies ou un sous-type diagnostiqué de cette maladie.

**9.** Utilisation du 5-ASA ou de l'un de ses sels ou esters acceptables pharmaceutiquement pour la fabrication d'un médicament pour le traitement des troubles inflammatoires de l'intestin, en particulier de la maladie de Crohn, de la colite ulcéreuse, d'une forme non classée de ces maladies ou d'un sous-type diagnostiqué de cette maladie, le médicament comprenant :

 des granulés enrobés individuellement, chaque granulé comprenant :

 - un noyau comprenant essentiellement de l'acide 5-aminosalicylique (5-ASA) (ou l'un de ses sels ou esters) et un adjuvant de sphéronisation acceptable physiologiquement, de préférence un dérivé de cellulose, en particulier de la cellulose microcristalline, et,
 - un enrobage enrobant le noyau, l'enrobage comprenant une matière formant barrière limitant la vitesse qui est une matière formant barrière limitant la vitesse de diffusion ou une matière formant barrière pouvant s'éroder, se dégrader et limitant la vitesse, de préférence un polymère semi-perméable, en particulier de l'éthycellulose ; et

 la majorité des granulés, de préférence plus de 80 % et mieux encore plus de 90 % des granulés étant essentiellement sphériques tels que définis par un rapport d'aspect (rapport de la longueur à la largeur) compris entre 1,00 et 1,25, de préférence entre 1,00 et 1,20 et mieux encore entre un 1,00 et 1,15 ; et

la majorité des granulés, de préférence plus de 70 %, et mieux encore plus de 90 % des granulés de la composition ayant des valeurs granulométriques dans l'intervalle allant de ≥ 0,5 à <1,4 mm, de préférence dans l'intervalle allant de ≥ 0,7 à ≤ 1,1 mm; et

la composition ayant les vitesses suivantes de dissolution in vitro [telles que mesurées dans un système modèle utilisant un fluide intestinal de simulation dans le système à pale N° 2 de la pharmacopée des Etats-Unis d'Amérique fonctionnant à 37°C à une vitesse d'agitation de 100 tours à la minute] :

a) entre 2 et 20 %, de préférence entre 5 et 15 % du 5-ASA total est libéré après 15 minutes dans le système modèle ;
b) entre 20 et 50 %, de préférence entre 25 et 45 % du 5-ASA total est libéré après 60 minutes dans le système modèle ;
c) entre 30 et 70 %, de préférence entre 40 et 60 % du 5-ASA total est libéré après 90 minutes dans le système modèle ;
d) entre 50 et 90 %, de préférence entre 55 et 80 % du 5-ASA total est libéré après 150 minutes dans le système modèle ;

entre 75 et 100 % du 5-ASA total est libéré après 240 minutes dans le système modèle.

Figure 1    Image Analysis of Spherical Granules (Batch 322202)

### Figure 2 Analysis of geometrical characteristica of spherical granules (Batch322202)

| No | Length | Breadth | Peri-meter | Aspect Ratio |
|---|---|---|---|---|
| 1 | 0.829 | 0.777 | 2.617 | 1.066 |
| 2 | 0.842 | 0.712 | 2.513 | 1.181 |
| 3 | 0.842 | 0.777 | 2.655 | 1.083 |
| 4 | 0.803 | 0.699 | 2.500 | 1.148 |
| 5 | 0.881 | 0.777 | 2.746 | 1.133 |
| 6 | 0.842 | 0.751 | 2.578 | 1.120 |
| 7 | 0.816 | 0.777 | 2.604 | 1.050 |
| 8 | 0.933 | 0.855 | 2.927 | 1.090 |
| 9 | 0.777 | 0.687 | 2.396 | 1.132 |
| 10 | 0.946 | 0.868 | 2.979 | 1.089 |
| 11 | 1.101 | 0.881 | 3.251 | 1.250 |
| 12 | 0.829 | 0.777 | 2.617 | 1.066 |
| 13 | 0.946 | 0.816 | 2.927 | 1.158 |
| 14 | 0.894 | 0.829 | 2.772 | 1.078 |
| 15 | 0.842 | 0.764 | 2.668 | 1.101 |
| 16 | 0.907 | 0.738 | 2.694 | 1.228 |
| 17 | 0.907 | 0.803 | 2.772 | 1.129 |
| 18 | 0.972 | 0.920 | 3.070 | 1.056 |
| 19 | 0.881 | 0.816 | 2.811 | 1.079 |
| 20 | 0.972 | 0.816 | 2.953 | 1.190 |
| 21 | 0.907 | 0.842 | 2.876 | 1.076 |
| 22 | 0.790 | 0.712 | 2.448 | 1.109 |
| 23 | 1.075 | 0.881 | 3.174 | 1.220 |
| 24 | 0.920 | 0.790 | 2.824 | 1.163 |
| 25 | 0.959 | 0.855 | 2.927 | 1.121 |
| Mean | 0.897 | 0.797 | 2.772 | 1.125 |
| St. Dev. | 0.081 | 0.061 | 0.221 | 0.056 |
| Max. | 1.101 | 0.920 | 3.251 | 1.250 |
| Min. | 0.777 | 0.687 | 2.396 | 1.050 |

| No | Length | Breadth | Peri-meter | Aspect Ratio |
|---|---|---|---|---|
| 1 | 0.894 | 0.829 | 2.811 | 1.078 |
| 2 | 0.933 | 0.855 | 2.927 | 1.090 |
| 3 | 0.933 | 0.855 | 2.902 | 1.090 |
| 4 | 0.855 | 0.803 | 2.694 | 1.064 |
| 5 | 0.881 | 0.803 | 2.746 | 1.096 |
| 6 | 0.712 | 0.661 | 2.267 | 1.078 |
| 7 | 0.894 | 0.842 | 2.824 | 1.061 |
| 8 | 0.751 | 0.661 | 2.319 | 1.137 |
| 9 | 0.829 | 0.764 | 2.617 | 1.084 |
| 10 | 0.803 | 0.764 | 2.539 | 1.050 |
| 11 | 0.946 | 0.855 | 2.953 | 1.106 |
| 12 | 1.023 | 0.868 | 3.135 | 1.179 |
| 13 | 0.855 | 0.777 | 2.668 | 1.100 |
| 14 | 0.816 | 0.751 | 2.513 | 1.086 |
| 15 | 0.842 | 0.816 | 2.720 | 1.031 |
| 16 | 0.881 | 0.803 | 2.772 | 1.096 |
| 17 | 0.946 | 0.868 | 2.915 | 1.089 |
| 18 | 0.803 | 0.751 | 2.578 | 1.068 |
| 19 | 0.920 | 0.842 | 2.876 | 1.092 |
| 20 | 0.842 | 0.751 | 2.578 | 1.120 |
| 21 | 0.868 | 0.829 | 2.759 | 1.046 |
| 22 | 0.907 | 0.881 | 2.915 | 1.029 |
| 23 | 0.881 | 0.816 | 2.811 | 1.079 |
| 24 | 0.855 | 0.790 | 2.746 | 1.081 |
| 25 | 0.907 | 0.829 | 2.837 | 1.093 |
| Mean | 0.871 | 0.803 | 2.737 | 1.085 |
| St. Dev. | 0.066 | 0.058 | 0.198 | 0.032 |
| Max. | 1.023 | 0.881 | 3.135 | 1.179 |
| Min. | 0.712 | 0.661 | 2.267 | 1.029 |

| No | Length | Breadth | Peri-meter | Aspect Ratio |
|---|---|---|---|---|
| 1 | 0.777 | 0.738 | 2.487 | 1.052 |
| 2 | 0.946 | 0.868 | 2.979 | 1.089 |
| 3 | 0.894 | 0.803 | 2.746 | 1.112 |
| 4 | 0.933 | 0.842 | 2.850 | 1.107 |
| 5 | 0.829 | 0.764 | 2.591 | 1.084 |
| 6 | 0.946 | 0.855 | 2.940 | 1.106 |
| 7 | 0.894 | 0.803 | 2.733 | 1.112 |
| 8 | 0.868 | 0.790 | 2.746 | 1.098 |
| 9 | 0.907 | 0.855 | 2.837 | 1.060 |
| 10 | 0.920 | 0.855 | 2.876 | 1.075 |
| 11 | 0.933 | 0.803 | 2.863 | 1.161 |
| 12 | 0.920 | 0.829 | 2.863 | 1.109 |
| 13 | 0.816 | 0.725 | 2.487 | 1.125 |
| 14 | 0.894 | 0.790 | 2.746 | 1.131 |
| 15 | 0.829 | 0.751 | 2.617 | 1.103 |
| 16 | 0.933 | 0.894 | 2.979 | 1.043 |
| 17 | 0.907 | 0.868 | 2.927 | 1.044 |
| 18 | 0.751 | 0.674 | 2.319 | 1.115 |
| 19 | 0.855 | 0.777 | 2.642 | 1.100 |
| 20 | 0.816 | 0.777 | 2.604 | 1.050 |
| 21 | 0.907 | 0.868 | 2.889 | 1.044 |
| 22 | 0.816 | 0.738 | 2.513 | 1.105 |
| 23 | 0.842 | 0.751 | 2.578 | 1.120 |
| 24 | 0.920 | 0.829 | 2.824 | 1.109 |
| 25 | 0.855 | 0.738 | 2.642 | 1.157 |
| Mean | 0.876 | 0.799 | 2.731 | 1.096 |
| St. Dev. | 0.055 | 0.056 | 0.176 | 0.033 |
| Max. | 0.946 | 0.894 | 2.979 | 1.161 |
| Min. | 0.751 | 0.674 | 2.319 | 1.043 |

Batch

Batch no.: 322202

| | Length | Breadth | Peri-meter | Aspect Ratio |
|---|---|---|---|---|
| Mean | 0.881 | 0.800 | 2.747 | 1.102 |
| St. Dev. | 0.068 | 0.058 | 0.197 | 0.044 |
| Max. | 1.101 | 0.920 | 3.251 | 1.250 |
| Min. | 0.751 | 0.674 | 2.319 | 1.029 |

EP 0 871 434 B2

Figure 3    Image Analysis of Spherical Granules (Batch
            437601)

## Figure 4 Analysis of geometrical characteristica of spherical granules (Batch 437601)

| No | Length | Breadth | Perimeter | Aspect Ratio | No | Length | Breadth | Perimeter | Aspect Ratio | No | Length | Breadth | Perimeter | Aspect Ratio |
|---|---|---|---|---|---|---|---|---|---|---|---|---|---|---|
| 1 | 0.725 | 0.674 | 2.319 | 1.076 | 1 | 0.687 | 0.609 | 2.111 | 1.127 | 1 | 0.842 | 0.777 | 2.642 | 1.083 |
| 2 | 0.803 | 0.751 | 2.526 | 1.068 | 2 | 0.842 | 0.777 | 2.655 | 1.083 | 2 | 0.868 | 0.790 | 2.668 | 1.098 |
| 3 | 0.712 | 0.661 | 2.241 | 1.078 | 3 | 0.946 | 0.829 | 2.902 | 1.140 | 3 | 0.881 | 0.816 | 2.772 | 1.079 |
| 4 | 0.946 | 0.842 | 2.953 | 1.123 | 4 | 0.933 | 0.868 | 2.927 | 1.074 | 4 | 0.777 | 0.687 | 2.370 | 1.132 |
| 5 | 0.959 | 0.868 | 2.966 | 1.104 | 5 | 0.803 | 0.725 | 2.513 | 1.107 | 5 | 0.764 | 0.648 | 2.306 | 1.180 |
| 6 | 0.920 | 0.868 | 2.876 | 1.059 | 6 | 0.816 | 0.764 | 2.565 | 1.067 | 6 | 0.907 | 0.751 | 2.720 | 1.206 |
| 7 | 0.868 | 0.790 | 2.720 | 1.098 | 7 | 0.777 | 0.674 | 2.345 | 1.153 | 7 | 0.997 | 0.907 | 3.109 | 1.100 |
| 8 | 0.933 | 0.881 | 2.992 | 1.058 | 8 | 0.894 | 0.842 | 2.811 | 1.061 | 8 | 0.842 | 0.777 | 2.591 | 1.083 |
| 9 | 0.842 | 0.803 | 2.694 | 1.048 | 9 | 0.738 | 0.583 | 2.189 | 1.266 | 9 | 0.790 | 0.738 | 2.513 | 1.070 |
| 10 | 0.907 | 0.803 | 2.850 | 1.129 | 10 | 0.816 | 0.712 | 2.422 | 1.145 | 10 | 0.868 | 0.803 | 2.733 | 1.080 |
| 11 | 0.842 | 0.764 | 2.642 | 1.101 | 11 | 0.790 | 0.751 | 2.526 | 1.051 | 11 | 0.933 | 0.855 | 2.902 | 1.090 |
| 12 | 0.829 | 0.738 | 2.552 | 1.122 | 12 | 0.790 | 0.777 | 2.565 | 1.016 | 12 | 0.816 | 0.712 | 2.487 | 1.145 |
| 13 | 0.933 | 0.829 | 2.915 | 1.125 | 13 | 0.829 | 0.687 | 2.500 | 1.207 | 13 | 0.855 | 0.803 | 2.707 | 1.064 |
| 14 | 0.790 | 0.764 | 2.526 | 1.033 | 14 | 0.751 | 0.699 | 2.370 | 1.074 | 14 | 0.842 | 0.777 | 2.617 | 1.083 |
| 15 | 0.790 | 0.751 | 2.539 | 1.051 | 15 | 0.803 | 0.687 | 2.396 | 1.169 | 15 | 0.816 | 0.777 | 2.591 | 1.050 |
| 16 | 0.868 | 0.764 | 2.681 | 1.135 | 16 | 0.816 | 0.764 | 2.591 | 1.067 | 16 | 0.907 | 0.855 | 2.863 | 1.060 |
| 17 | 0.816 | 0.738 | 2.539 | 1.105 | 17 | 0.738 | 0.661 | 2.306 | 1.117 | 17 | 0.712 | 0.648 | 2.215 | 1.100 |
| 18 | 0.803 | 0.738 | 2.487 | 1.087 | 18 | 0.790 | 0.751 | 2.539 | 1.051 | 18 | 1.010 | 0.868 | 3.057 | 1.164 |
| 19 | 0.842 | 0.764 | 2.604 | 1.101 | 19 | 0.803 | 0.712 | 2.422 | 1.127 | 19 | 0.881 | 0.842 | 2.850 | 1.046 |
| 20 | 0.751 | 0.687 | 2.370 | 1.094 | 20 | 0.868 | 0.790 | 2.707 | 1.098 | 20 | 0.790 | 0.712 | 2.461 | 1.109 |
| 21 | 0.816 | 0.738 | 2.513 | 1.105 | 21 | 0.751 | 0.674 | 2.306 | 1.115 | 21 | 0.881 | 0.816 | 2.720 | 1.079 |
| 22 | 0.842 | 0.764 | 2.655 | 1.101 | 22 | 0.712 | 0.648 | 2.241 | 1.100 | 22 | 0.790 | 0.738 | 2.461 | 1.070 |
| 23 | 0.829 | 0.751 | 2.565 | 1.103 | 23 | 0.984 | 0.855 | 3.005 | 1.151 | 23 | 0.803 | 0.764 | 2.552 | 1.050 |
| 24 | 0.790 | 0.712 | 2.435 | 1.109 | 24 | 0.842 | 0.764 | 2.668 | 1.101 | 24 | 0.816 | 0.790 | 2.630 | 1.032 |
| 25 | 0.777 | 0.622 | 2.306 | 1.250 | 25 | 0.816 | 0.764 | 2.565 | 1.067 | 25 | 0.894 | 0.829 | 2.772 | 1.078 |
| Mean | 0.837 | 0.763 | 2.619 | 1.099 | Mean | 0.813 | 0.735 | 2.526 | 1.109 | Mean | 0.851 | 0.779 | 2.652 | 1.093 |
| St. Dev. | 0.067 | 0.065 | 0.213 | 0.042 | St. Dev. | 0.071 | 0.073 | 0.228 | 0.054 | St. Dev. | 0.069 | 0.065 | 0.214 | 0.043 |
| Max. | 0.959 | 0.881 | 2.992 | 1.250 | Max. | 0.984 | 0.868 | 3.005 | 1.266 | Max. | 1.010 | 0.907 | 3.109 | 1.206 |
| Min. | 0.712 | 0.622 | 2.241 | 1.033 | Min. | 0.687 | 0.583 | 2.111 | 1.016 | Min. | 0.712 | 0.648 | 2.215 | 1.032 |

**Batch**

| | Length | Breadth | Perimeter | Aspect Ratio |
|---|---|---|---|---|
| Mean | 0.834 | 0.759 | 2.599 | 1.100 |
| St. Dev. | 0.070 | 0.069 | 0.222 | 0.046 |
| Max. | 1.010 | 0.907 | 3.109 | 1.266 |
| Min. | 0.712 | 0.648 | 2.215 | 1.016 |

Batch no.: 437601

Figure 5.    Figure of the Specifications Wanted for the In Vitro
             Release of 5-ASA.

In Vitro Dissolution Rate Specifications as Indicated by
the extreme as well as prefered upper and lower limit

Figure 6    In-vitro    Dissolution    Profiles    of    conventional
granules (Mean of six batches).

In Vitro DissolutionProfile of Conventional Granules
Tested in simulated Intestinal Fluid Model

Figure 7.. In-vitro Dissolution Profiles of spherical granules (Batch 322202).

In Vitro DissolutionProfile of Spherical Granules (Batch 3222202) Tested in simulated Intestinal Fluid Model

Figure 8.    In-vitro Dissolution Profiles of spherical granules (Batch 437601).

In Vitro DissolutionProfile of Spherical Granules (Batch 437601) Tested in simulated Intestinal Fluid Model

Figure 9. Comparison of In Vitro Release Profiles of Spherical Granules and Conventional Granules

Comparison of In Vitro Dissolution profiles of Two Batches of Spherical Granules and Conventional Granules

Figure 10    Gastric Emptying Time and Colon Arrival Time of
             Spherical Granules.

## Disposition data of Spherical Granules (Batch 437601)

| | Gastric Emptying Time (mins) | | Colon Arrival Time (mins) | |
|---|---|---|---|---|
| | $T_{50\%}$ | $T_{100\%}$ | $T_{50\%}$ | $T_{100\%}$ |
| 1 | 22 | 60 | 100 | 121 |
| 2 | 33 | 124 | 308 | 727 |
| 3 | 10 | 83 | 260 | 513 |
| 4 | 25 | 65 | 200 | 277 |
| 5 | 38 | 124 | 253 | 261 |
| 7 | 17 | 73 | 292 | 469 |
| 8 | 13 | 65 | 154 | 172 |
| 9 | 15 | 30 | 82 | 180 |
| Mean | 22 | 78 | 206 | 340 |
| SD | 10 | 32 | 86 | 210 |
| RSD % | 46 | 41 | 42 | 62 |
| Median | 20 | 69 | 227 | 269 |

Figure 11.   Plasma Concentration Curve of 5-ASA After Dosing
with Spherical Granules (Batch similar to 322202)
Containing 1000 mg 5-ASA (Steady State)

Plasma Concentration Curves of 5-ASA in Healthy Volunteers
After Intake of Spherical Granules 1000 mg Single Dose.
(Mean, N=8)

Spherical Granules (Batch 437601)

Figure 12. Plasma Concentration Curve of 5-ASA After Dosing with Spherical Granules (Batch 437601) Containing 1000 mg 5-ASA ( Single Dose Study)

Plasma Concentration Curves of 5-ASA in Healthy Volunteers After Intake of Spherical Granules 1000 mg Single Dose. (Mean, N=8)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4496553 A
- US 4980173 A, Halskov

- WO 9428911 A
- WO 9107949 A

**Non-patent literature cited in the description**

- **BECHGAARD, H.** *Acta Pharmaceutica Technologica,* 1982, vol. 28 (2
- **CHRISTENSEN, L.A. et al.** *Br, J. Clin. Pharmac.,* 1987, vol. 23, 365-369

- **FÄLLINGBORG, J. et al.** *Aliment. Pharmacol. therap.,* 1989, vol. 3, 605-613